# EUROPEAN PATENT APPLICATION

(11) **EP 4 609 907 A1**
(43) Date of publication of application: **03.09.2025**
(21) Application number: 24161036.9
(22) Date of filing: 02.03.2024
(51) Int. Cl.: A61M 25/10, A61M 25/098, A61F 2/958, A61M 25/00, A61M 25/01

(54) **BALLOON CATHETER SUCH AS PTA/PTCA - CATHETER**

(71) Applicant: SMD Swiss Medical Devices, 8222 Beringen (CH)
(72) Inventor: BEHR, Jean-Marc, 8200 Schaffhausen (CH); NASSISI, Quentin, 1299 Crans (CH); ZWAHLEN, Willi, 6760 Betschdorf (FR)
(74) Representative: BOVARD AG

(57) **Abstract**

The present invention is directed to a balloon catheter (1) such as a PTA-catheter or a PTCA-catheter comprising an inflatable balloon (20) defining an inner balloon lumen (20) with a distal end portion (22), cylindrical portion (26) and a proximal end portion (23), an inner shaft (20) passing through the inner balloon lumen (21), the inner shaft (10) including an inner layer (12), an outer layer (14), and a support body (16), the support body (16) is embedded in the outer layer (14), extends at least over at least a part of the inner shaft (10), the part being positioned within the inner balloon lumen (21), and at least a radiopaque marker (50) including a material with radiopaque property located within the inner shaft (10).

## Description

### Technical Field

The invention relates to a balloon catheter, in particular a PTA/PTCA-catheter, which is configured for treatments for coronary diseases, especially in percutaneous transluminal angioplasty (PTA) procedures and percutaneous transluminal coronary angioplasty (PTCA) procedures.

### Background of the invention

The invention relates to balloon catheters such as PTA-balloon catheter or PTCA-balloon catheter for use in the performance of percutaneous transluminal angioplasty (PTA) procedures including percutaneous transluminal coronary angioplasty (PTCA), coronary angioplasty and valvuloplasty. Angioplasty techniques typically involve the use of a balloon catheter which is advanced over a guide wire so that the balloon is positioned adjacent to the stenotic lesion. The balloon is inflated to widen an occluded, stenotic, or narrowed region of an artery such as coronary arteries, peripheral arteries, and veins, which are transporting or carrying blood. Alternatively, the balloon catheter can be as well used for introducing and positioning a stent.

During a cardiological intervention, a guiding catheter is inserted into the patient, commonly entering through the femoral or radial artery, and advanced towards the site of treatment, e.g. placed into the ostium in case of a coronary artery intervention. Afterwards, a guide wire is inserted through the guiding catheter and advanced to the site of treatment. Subsequently, a catheter is slid over the guide wire to be moved to the lesion.

Typically, a balloon catheter comprises a balloon at a distal end of a catheter shaft or inner shaft which can be slid over the guide wire. The balloon has a proximal section and a distal section and an inflatable section therebetween, wherein the proximal section and/or the distal section are affixed to the catheter shafts. The balloon is freely inflatable and deflatable by adjusting the internal pressure of a pressure fluid supplied through a lumen, the inflation lumen, provided inside the catheter shaft along the longitudinal direction of the catheter shaft. Balloon catheters can be configured as over-the-wire balloon catheter or as rapid exchange balloon catheter in which the guide wire lumen extends only in the distal part of the catheter in order to allow easy exchange of the balloon catheter.

In order to manoeuvre the balloon catheter to appropriate locations in the coronary vascular tree, the dilatation catheter must be small in diameter. Furthermore, it should be highly flexible, so it can easily bend and be guided through the artery system but as well stiff enough to be pushable and trackable. The catheter shaft and the guide wire are however weak components in the equipment and can cause potentially serious complication due to occasional damage or breakage leaving a small piece in the artery of the patient. Once in position across the lesion, the flexible, expandable balloon whether compliant, semi- or non-compliant is inflated up to a rated burst pressure to transfer sufficient stress to the atherosclerotic plaque of the lesion to break it. The balloon is then deflated to a small profile, so that the dilatation catheter can be withdrawn from the patient's vasculature.

In order to assist guidance and positioning and to confirm the insertion length of the catheter in the body and/or the position of the balloon and/or the catheter tip, at least one marker band is provided that is highly visible under fluoroscope or X-ray. This marker band is often made of a metal material such as gold, platinum, tungsten, iridium, or alloys thereof and is formed in a rigid thin tubular shape swaged onto an inner or outer shaft of the balloon catheter. Such rigid metal markers affect the flexibility of the catheter, locally stiffening the catheter shaft, imparts an undesirable discontinuity in the catheter profile thereto and can present problems to passing the catheter through a lesion.

When fixing the metal marker band for example at the catheter shaft, the typically short, thin-walled tube is mechanically crimped, swaged, welded, or glued with instant adhesive. In particular the crimping is often done by hand, which does not provide optimal results. Furthermore, these fixing methods result in poor flexibility such that the catheter cannot pass through blood vessel and create a weak portion. Forming a recess, i.e. for crimping or swaging, in the inner shaft for the metal marker band eliminates the step difference but since the wall thickness becomes thinner, the strength of the inner shaft decreases.

To overcome the local stiffness of known metal marker bands US6520934B1 discloses a catheter assembly with at least one segmented marker band comprising radiopaque material or as a coiled band of a radiopaque material, formed by winding a wire or hollow tube filled with radiopaque material. This coiled band does not extend to the tip of the catheter such that the catheter does not provide sufficient strength. The flexible marker may be affixed with the shaft by an adhesive, by swaging, by crimping, by soldering or by spring-tension fit against the shaft. These flexible markers may damage, pinch, or incise the inner shaft.

Another approach is disclosed in JP 20002204832A. This document describes coiled markers made of a radiopaque metal attached to a catheter tube, in particular the guide wire tube. Both ends of the coiled marker are attached to fixed tube parts of the catheter tube with tapered outer circumferences by heat welding, adhesive or other means such that no step is formed between the parts of the catheter tube. No further design of a coiled marker is disclosed such as a coil coated or plated radiopaque material.

To provide a catheter not only with high flexibility but as well with a certain stiffness WO 9411053 discloses a catheter known as over-the-wire type balloon catheter comprising a dilatation balloon affixed to its distal end and an elongated shaft forming a lumen. The shaft is formed of a proximal hypotube extending at least half the length of the catheter and a distal spring coil attached to the hypotube and surrounded by a jacket forming the remainder of the shaft. A portion of the spring coil or the entire spring coil can be fabricated of a radiopaque material to make it visible under a fluoroscope.

In document EP 2 719 418 A1 a balloon catheter is disclosed comprising a balloon and an inner shaft passing through an inner lumen of the balloon, including an inner layer, an outer layer, and a support body, for example a coil structure, which is embedded in the outer layer and extends the entire length of the balloon inner shaft. Radiopaque markers are provided either by the support body having radiopaque properties or by a radiopaque marker formed as a band swaged onto the outer surface an inner tube and located in a more inner position in the radial direction than the support body.

It is therefore an object of the present invention to improve balloon catheters. It is another object of the present invention to provide balloon catheter that are significantly strengthened against breakage while retaining, showing increased flexibility, pushability, steerability, kink resistance, and/or trackability. It is another object of the present invention to provide a balloon catheter with at least one radiopaque marker which minimizes or totally prevents steps in diameters, weakened portions and is easily to attach to the catheter right at the predetermined position.

### Summary of the invention

According to an aspect of the present invention, there is provided a balloon catheter comprising:
- an inflatable balloon defining an inner balloon lumen with a distal end portion and a proximal end portion,
- an inner shaft passing through the inner balloon lumen, the inner shaft including an inner layer, an outer layer, and a support body,
- the support body is embedded in the outer layer and extends over at least a part of the inner shaft, the part being positioned within the inner balloon lumen, the support body is a coil-shaped body, and
- at least a radiopaque marker including a material with radiopaque property located within the inner shaft.

A balloon catheter such as a PTA-catheter or PTCA-catheter is an elongated intracorporeal device for percutaneous transluminal angioplasty procedures, but as well for stent deployment, use for application of therapeutic drugs (DCB) and similar procedures. According to the invention, the balloon catheter comprises an inflatable or expandable and deflatable balloon having a distal end portion, a proximal end portion and in between a substantially cylindrical portion extending from a distal end to a proximal end thereof, and an inner shaft. The inner shaft has in radial direction an outer layer, a support body embedded in the outer layer and an inner layer. Therefore, the inner shaft can be configured as a multilayer component comprising as well more than the outer layer, the support body, and the inner layer. Furthermore, the inner layer and the outer layer may be made of the same material or different materials. The inner shaft can be described as an elongated member with a distal end and a proximal end in longitudinal direction. Therefore, structurally the balloon catheter comprises the flexible inner shaft between the proximal end and the distal end thereof and defines one or more tubular passages or lumens extending through part or all of the catheter inner shaft. Such lumens often have one or more openings, referred as ports.

It is desirable to provide a balloon catheter having an optimal combination of some of the features such as flexibility, pushability, trackability, crossability and others. Flexibility may relate to bending stiffness of the balloon catheter in a particular region or over the entire length or may relate to material stiffness of the components. Pushability may relate to column strength of a device or system along a selected path. Trackability may refer to a capability of the device to successfully follow a desired path. The trackability and crossability are furthermore affected by the surface properties and the profile along the catheter. Crossability may relate to reaching the desired site, e.g. the ease of correctly placing the balloon within the lesion with little or no friction or resistance.

The balloon catheter of the present invention may be configured as a rapid exchange catheter and is capable of advancement into the vascular system of a patient along a pre-positioned guide wire. Typically, the guide wire exits an internal catheter lumen through a proximal guide wire port and extends in parallel along the outside of the catheter proximal portion. The guide wire occupies a catheter lumen extending only through a distal portion of the catheter. Therefore, the entire catheter and guide wire assembly is typically contained within a lumen of a guiding catheter, which surrounds and guides the balloon catheter or stent delivery system to the desired site. This system is advantageous over the over-the-wire type catheters, because the guide wire can be left in place and the catheter exchanged rapidly due to a short distal piece required being slid over the wire.

The balloon may be attached to the inner shaft. It is advantageously to form the balloon and the outer layer of the inner shaft of compatibles materials, for example thermoplastic polymers, so that the outer layer of the inner shaft can be thermally bonded, e.g. heat welded, to the balloon. In the case the balloon and the inner shaft of the catheter are made of materials that cannot be thermally bonded together, it is necessary to bond the balloon and the inner shaft my means of adhesives or other means which can result that these bonding parts become rigid.

Advantageously the inner shaft of the balloon catheter is reinforced with the support body. The support body reinforces at least part of the inner shaft to prevent the inner shaft tendency to kink and therefore improves the level of kink resistance and the pushabilty of the balloon catheter without much affecting the flexibility.

According to the invention, the support body may be formed as a coiled-shaped body embedded in the inner shaft, preferably in the outer layer. The coiled-shaped body may comprise a coil wire wrapped around the inner layer of the inner shaft. The coil wire may have any suitable form. Alternatively, the support body may be formed as a braid-shaped body surrounding the inner layer of the inner shaft, the braid body itself being embedded in the outer layer of the inner shaft.

The coil wire can be configured as a flat wire with a rectangular cross section or a chamfered-rectangular cross section. A flat wire has the advantage that the wall thickness of the inner shaft increases only minimally, the pressing performance of the balloon catheter is improved and in case parts of the coil-shaped body are configured as a radiopaque marker, the visible surface is increased. Alternatively, the coil wire may have a round cross section with the advantage that the pliability of the balloon catheter could be improved.

The inner shaft may comprise at least two materials, in particular the material of the coil-shaped body and the material of the layer of the inner shaft. The inner layer and the outer layer of the inner shaft may be made of the same material or different materials. In particular, the outer layer of the inner shaft is formed in contact with the inner layer such that an inner surface of the outer layer is in contact with the coil-shaped body and with the inner layer between turns of the coiled-shaped body.

It is advantageous that at least the outer layer of the inner shaft comprises a thermoplastic material such that it is appropriate to heat-welding, forms smooth surfaces, and/or reduces formation of blood clots thereon. By forming the inner shaft including the outer layer and the inner layer, the support body can have a variable diameter such to affect the flexibility of the balloon catheter. The adjustable position of the support body in radial direction is not particularly limited.

The support body configured as the coiled-shaped body is disposed on the inner shaft. However, the disposing position of the support body along the length of the inner shaft is not particularly limited. The support body may be disposed only within the inner balloon lumen, may extend to the attaching portions of the balloon with the inner shaft or an outer shaft or may extend beyond these attaching portions, preferably over at least the cylindrical portion or over almost an entire length of the inner shaft, in particular from the distal end to the proximal end thereof.

At the distal end of the inner shaft at least the outer layer can extend beyond the end of the support body forming a tapered distal tip.

The balloon catheter of the invention includes radiopaque markers to aid in imaging the catheter while it is in the vascular system of a patient. In particular, the radiopaque marker indicates the cylindrical portion of the balloon that is important to not dilate the healthy part of the vessel, which may be leading to intima injury and therefore creates restenosis in an originally healthy part of the artery. Typically, percutaneous devices such as balloon catheters utilize one or more radiopaque markers, in order for the operator of this device to ascertain its position and orientation under X-ray fluoroscopy imaging. Known radiopaque markers are often provided as a metal band crimped onto the catheter. Various metals are highly radiopaque materials but are limited in flexibility and have the risk of causing abrasion. Polymers are often not sufficiently radiopaque to provide satisfactory results for X-ray based examinations.

In some embodiments of the invention, the radiopaque marker is provided by the support body such that at least a part of the support body, either configured as a coiled-shaped body or a braid-shaped body is made of a material with radiopaque property forming at least one radiopaque marker, wherein the support body is fully embedded into the inner shaft. This material may be platinum, tungsten, gold, iridium, silver, or alloys thereof but is not limited to these materials. This part of the support body with radiopaque property is at least located between the distal end of the inner shaft, in particular the distal tip, and a predetermined portion of the balloon catheter for example in the case of a rapid exchange balloon catheter proximal to the Rapid Exchange (RX) port or side port. Preferably, the radiopaque part of the support body indicates the position and length of the balloon, in particular the cylindrical length thereof.

According to another embodiment of the invention, the radiopaque marker may be formed as a coating or plating of at least a part of the support body, configured as a coiled-shaped body or in other form, with a material with radiopaque property. According to this embodiment the radiopaque marker is provided such that the outer diameter of the inner shaft is not or only minimal increased. Therefore, the thickness of the coating or plating is selected that still the support body is fully embedded into layers of the inner shaft. The radiopaque coating or plating of at least one part of the coiled-shaped body has a suitable thickness enough to provide good contrast by imaging, corresponding to radiopacity values achievable with greater than 6 mm aluminium. Furthermore, being biomedically compatible, sufficiently ductile and showing resistance against strains. The coating or plating can be applied to predetermined more than one part of the support body such that the position and/or length of the balloon, preferably of the position of the inner balloon lumen, can be detected by X-ray fluoroscopy imaging.

Preferably, the support body configured as the coil-shaped body may be made of an elastic metal or alloy such as a stainless steel, Ni-Ti alloy, known as nitinol, or similar materials and then the coating is precisely applied to specific areas of the coiled-shaped body. Alternatively, the coiled-shaped body may be formed from a polymer and the radiopaque material coating is deposited without much affecting the flexibility. The material for the coating or plating may be a radiopaque metal material for example but not limited to tantalum, gold, platinum, iridium, tungsten, other radiopaque materials, or combinations thereof. The coating or plating of the coil-shaped body may also include radiopaque particles with micro or macro size.

However, for coating or plating other materials showing radiopaque property can also be used. Such alternative materials include for example material with filled polymer particles having radiopaque properties. For example, this coating material can be a tungsten filled thermoplastic elastomer such as Polyether-Block-Amide. The coating or plating should achieve secure attachment, stability and necessary radiopacity while maintaining mechanical properties of the coiled-shaped body.

According to some embodiments of the invention, at least one radiopaque marker is provided by part of the outer layer of the inner shaft including a radiopaque material. For example, part of the outer layer provided at the outer circumferential of the support body may be made of a polymer that is filled or doped with a suitable radiopaque agent. Such radiopaque marker material may be formed by blending a polymer with a powdered, radiographically dense material such as tungsten for example and then applying the composition to form the outer layer. The local application of a radiopaque doped polymer to an already coated or plated coil-shaped body may further enhance the radiopacity.

According to another embodiment, at least one radiopaque marker is configured as a ring with an inner diameter equal or greater than an outer diameter of the support body such as to be slipped over the support body and embedded in the outer layer. Therefore, the ring can be put on place along the support body easily and through the embedding in the outer layer no or minimally protruding parts of the catheter disturbs the implementation of the catheter.

The one or more radiopaque markers may be disposed on the inner shaft, preferably on the support body and/or the outer layer. For example, one radiopaque marker extends over the entire cylindrical portion of the inner balloon lumen. In another embodiment, a first radiopaque marker portion is located at the distal end of the cylindrical portion of the inner balloon lumen and a second radiopaque marker portion is located at the proximal end of the cylindrical portion of the inner balloon lumen. Preferably the first radiopaque marker and the second radiopaque marker are located such to indicate the distal end and the proximal end of the substantially cylindrical portion of the inner balloon lumen. Alternatively, one radiopaque marker is located either at the distal end of the cylindrical portion of the inner balloon lumen, at a proximal end of the cylindrical part of the inner balloon lumen, at a centre of the cylindrical part of the inner balloon lumen or at any position within the cylindrical portion of the inner balloon lumen.

In some embodiments of the invention, the material with radiopaque property is metallic, for example but not limited to platinum, gold, iridium, tungsten, tantalum, or an alloy thereof.

Materials of the radiopaque marker may include metal particles from a material having high radiopaque properties against X-rays fluoroscopy imaging, such as but not limited to platinum, tungsten, gold, iridium, silver, and alloys thereof.

According to the invention, the radiopaque marker is not exposed on the outer surface of the outer layer of the inner shaft but is located at a more inner position in the radial direction than the outer surface of the outer layer. Since the radiopaque marker is embedded within the walls of the inner shaft, the risks of balloon rupture and improper balloon pleating or folding are reduced.

### Brief description of the drawings

For a more complete understanding of the invention and the advantages thereof, exemplary embodiments of the invention are explained in more detail in the following description with reference to the accompanying figures, in which like reference characters designate like parts and in which:
Fig. 1 is a schematic perspective view of a balloon catheter;
Fig. 2 is a schematic perspective view of a distal end of the balloon catheter of a first embodiment;
Fig. 3 is a schematic detail view of a coiled-shaped body with a partially disposed coating of a material with radiopaque property;
Fig. 4 is a schematic perspective view of a distal end of the balloon catheter of a second embodiment;
Fig. 5 is a schematic perspective view of a distal end of the balloon catheter of a third embodiment;
Fig. 6 is a schematic detail view of an inner shaft of the balloon catheter in a fourth embodiment;
Fig. 7 is a schematic detail view of a distal end of the balloon catheter of a fifth embodiment;

### Preferred embodiments of the invention

The accompanying drawings are included to provide a further understanding of the present invention and are incorporated in and constitute a part of a specification. The figures illustrate particular embodiments of the invention and together with the description serve to explain the principles of the invention. Other embodiments of the invention and many of the attendant advantages of the invention will be readily appreciated, as they become better understood with reference to the following detailed description.

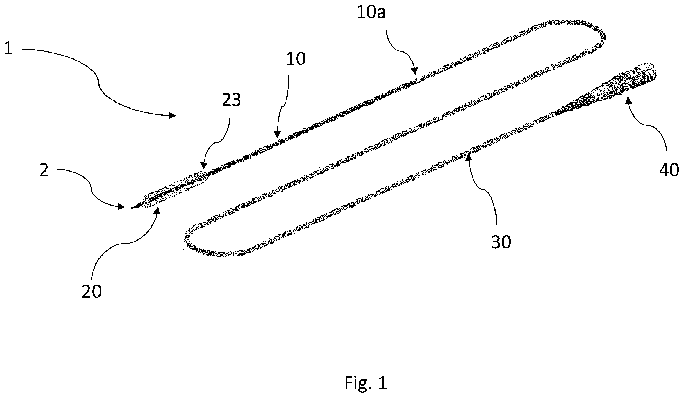

Fig. 1 schematically shows a balloon catheter 1 such as a PTA-catheter or a PTCA-catheter of the present invention including an inner shaft 10 forming a distal part or distal shaft of the balloon catheter 1, a balloon 20, shown in an inflated state, which is attached to a distal portion of the inner shaft 10, a proximal shaft 30 also mentioned as hypotube or outer shaft of the balloon catheter 1 attached to a proximal end of the inner shaft 10 and a hub 40 which is attached to a proximal end of the proximal shaft 30. Different designs of the proximal shaft 30 are known depending on a proximal attachment point of the proximal shaft 30 with either a RX-port 10a (rapid exchange port), the hub 40 or any position between the RX-port 10a and the hub 40. In the second case the proximal shaft 30 or outer shaft can be seen as the hypotube or at least both are of the same material.

In the case of a rapid exchange balloon catheter, the proximal shaft 30 is provided at least partly as a rigid shaft or hypotube and includes a proximal shaft guide wire port or the RX-port 10a (rapid exchange port) located proximal to a proximal end portion 23 of the balloon 20. The inner shaft 10 forms a hollow tube made of polymeric material and defines an inner lumen (not shown). The inner shaft 10 includes an open distal shaft guide wire port at the distal tip 2 of the balloon catheter 1 and stretches along a part of the length of the balloon catheter 1. The inner lumen of the inner shaft 10 is provided allowing the passage of the guide wire from the RX-port 10a to the inner shaft guide wire port. The combination of the inner lumen which is open at the distal end and an access opening in form of the RX-port 10a allows the passage of the guide wire out or into the inner lumen such that the balloon catheter 1 can be used as a rapid exchange catheter.

In order to inflate the balloon 20, an inflation lumen (not shown), which communicates with the inside of the balloon 20 and is separate from the inner lumen of the inner shaft 10 can be provided extending from an inflation port at the proximal end of the hub 40 through the inside of the proximal shaft 30 and between the inner wall of the proximal shaft 30 and the outer wall of the inner shaft 10. Different designs are possible such as a coaxial configuration of the inner lumen and the inflation lumen or a biaxial configuration.

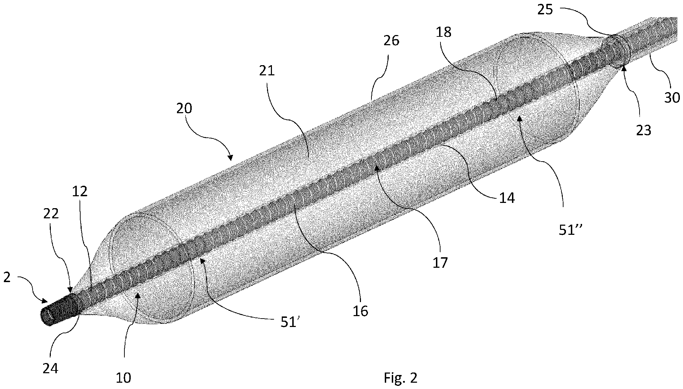

As shown in Figure 2, the balloon 20 is arranged on the inner shaft 10 and extends from a distal end portion 22 to the proximal end portion 23 defining an inner balloon lumen 21. The inner shaft 10 passes through the inner balloon lumen 21 and is connectable to the balloon 20 at least at the distal end portion 22. At the proximal end portion 23, as well seen as a proximal balloon neck, the balloon 20 can be attached to the proximal shaft 30, such that the proximal end portion 23 of the inner shaft 10 is not connected to the balloon 20. The inner shaft 10 is configured as a multi-layer structure comprising at least an inner layer 12, an outer layer 14 and a support body 16. The inner layer 12 forms the inner lumen of the inner shaft 10. The outer layer 14 forms an outer periphery or outer wall of the inner shaft 10 and covers an outer surface of the inner layer 12 and of the support body 16. Furthermore, the inner shaft 10 includes at least one radiopaque marker 50, shown in Fig. 2 are a first radiopaque marker portion 51' and a second radiopaque marker portion 51", to indicate and confirm the position of the balloon catheter 1, in particular of the balloon 20, in the body.

The inner layer 12 and/or the outer layer 14 can be made of a polymer. The inner layer 12 may be made of a polymer that can be bonded thermally, for example by heat-welding, mechanically or through adhesive to the outer layer 14 of the inner shaft 10. The outer layer 14 can be made of the same or a different polymer of the inner layer 12 such that they form together a firm laminate structure. Optionally, the inner layer 12 and/or the outer layer 14 can be heat-welded to form a distal port 24 or distal weld 24 of the balloon 20. The balloon 20 and the inner shaft 10 are heat-welded to each other at the distal end portion 22, described as well as the distal balloon neck, and the balloon 20 is connected at a proximal part 25 or proximal weld 25 of the balloon 20 with the proximal shaft 30.

The support body 16 is made of a material and has a form to achieve improvement of the strength as well as other mechanical properties of the inner shaft 10. For example, as an appropriate material superelastic alloy, or stainless steel, a high rigidity resin material, etc. can be used.

The thicknesses of the inner layer 12 and/or the outer layer 14 can be appropriately determined depending on the intended use of the balloon catheter 1.

Fig. 2 shows a distal part of the balloon catheter 1 according to a first embodiment with the inner shaft 10 including the inner layer 12, the outer layer 14 and the support body 16 extending through the inner balloon lumen 21 and connected to the balloon 20 at the distal end portion 22, for example by heat-welding. The inner balloon lumen 21 is limited by the distal end portion 22 and by the proximal end portion 23, between which the balloon 20 includes a substantially cylindrical portion 26.

As shown in Fig. 2, the support body 16 is designed as a coil-shaped body 17, which is formed by a rectangular wire arranged onto the outer surface of the inner layer 12 of the inner shaft 10 and embedded in the outer layer 14 thereof. Other embodiments of the support body 16 and of the coil-shaped body 17 are possible. The length of the support body 16 in longitudinal direction of the balloon catheter 1 can be the same as the entire length of the inner shaft 10 from the distal tip 2 to a determined position for example the position of the RX-port 10a of the balloon catheter 1 or shorter than that. In the latter case, the support body 16 at least needs to extend over the cylindrical portion 26 of the balloon 20. Therefore, the support body 16 may be disposed only within the inner balloon lumen 21, in particular the cylindrical portion 26 of the inner balloon lumen 21, may extend to the distal end portion 22 and/or the proximal end portion 23 or may extend beyond these portions 22, 23 to improve the pressing performance of the balloon catheter 1.

The coil-shaped body 17 can be formed from a rectangular wire or a round wire in a previous forming step and then placed onto the inner layer 12 of the inner shaft 10. Alternatively, the coil-shaped body 17 may be wound around the outer surface of the inner layer 12 of the inner shaft 10 into a coil shape. The coil-shaped body 17 is provided in an embedded state in the inner shaft 10, in particular is embedded in the outer layer 14 of the inner shaft 10 such that the inner layer 12, the support body 16 configured as the coil-shaped body 17 and the outer layer 14 forms an integral structure such that the inner shaft 10 does not have any gaps or diameter steps on the outer surface.

According to the first embodiment shown in Fig. 2, at least one radiopaque marker 50 is provided, in this case formed as a first radiopaque marker portion 51' and a second radiopaque marker portion 51" indicating the distal end portion 22 and the proximal end portion 23 of the inner balloon lumen 21 respectively, in particular the distal end and the proximal end of the cylindrical portion 26 of the inner balloon lumen 21. According to this first embodiment, the radiopaque marker 50 is formed as a coating or plating 18 on determined parts of the coil-shaped body 17. The coating or plating 18 is applied in the respective regions of the support body 16, for example the coated or plated regions extend over part of the length of the support body 16, for example over several turns of the coil-shaped body 17. The material for the coating or plating shows radiopaque property either as being of a radiopaque metal material or as being a material including radiopaque particles. Preferably, the first radiopaque marker portion 51' and the second radiopaque marker portion 51" are provided along parts of the length of the cylindrical portion 26 of the balloon 20 such that one end of the first radiopaque marker portion 51' indicates a distal end of the cylindrical portion 26 and the proximal end of the second radiopaque marker portion 51" indicates a proximal end of the cylindrical portion 26.

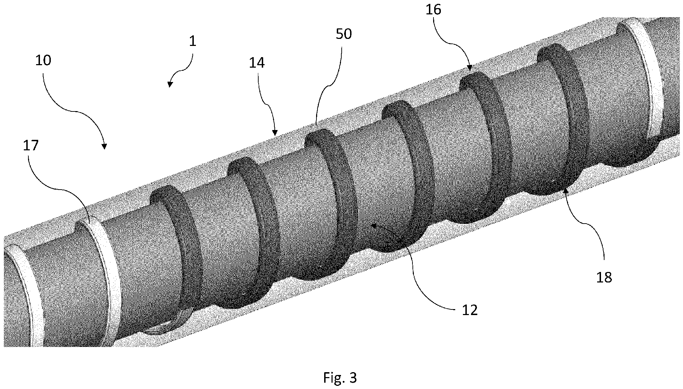

The coated region or regions may extend from the distal end portion 22 to the distal end of the cylindrical portion 26 and/or from the proximal end portion 23 to the proximal end of the cylindrical portion 26 of the inner balloon lumen 21 such that the uncoated portion represents the length of the cylindrical portion 26 of the inner balloon lumen 21. Therefore, the one or more radiopaque markers 50 are located between the distal tip 2 and the RX-port 10a. Fig. 3 shows in a schematic perspective view a detail of the distal end part of the balloon catheter 1 of the first embodiment. The support body 16, designed as the coil-shaped body 17 wound around the inner layer 12 of the inner shaft 10 is embedded in the outer layer 14. At least one part of the coil-shaped body 17 is coated or plated with radiopaque material to provide the radiopaque marker 50, in particular either with a radiopaque metal or a polymer including radiopaque metal particles. The coated radiopaque material forms the coating or plating 18 with a thickness of approximately 10 µm to 100 µm. According to this embodiment of the invention the radiopaque marker 50 is part of the inner shaft 10. Thus, no external radiopaque markers would be required that otherwise would for example have to be printed on the inner shaft 10, crimped or heat shrink welded causing protruding parts in radial direction such as a diameter step and/or complicated connecting processes.

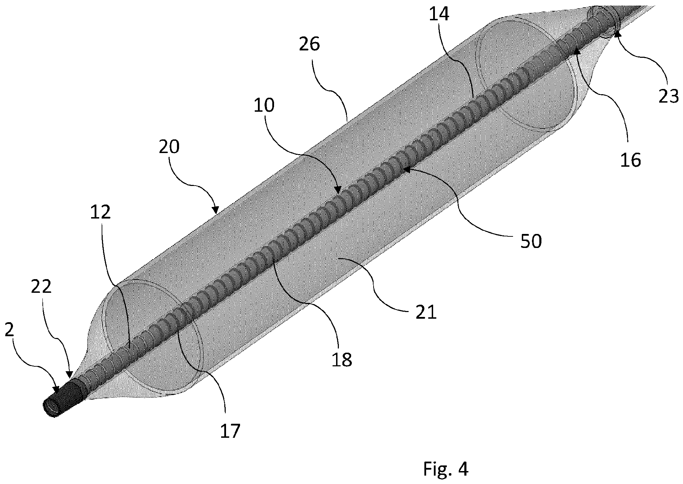

Alternatively, the coated region of the support body 16 may extend over part of the length of the coil-shaped body 17 corresponding to the length of the cylindrical portion 26 of inner balloon lumen 21, as shown in a second embodiment in Fig. 4.

Fig. 4 shows a second embodiment of the invention. According to this embodiment, the radiopaque marker 50 is configured as a coating or plating 18 of the coil-shaped body 17, configured as the support body 16, extending over part of the length thereof representing the length of the cylindrical portion 26 of the inner balloon lumen 21.

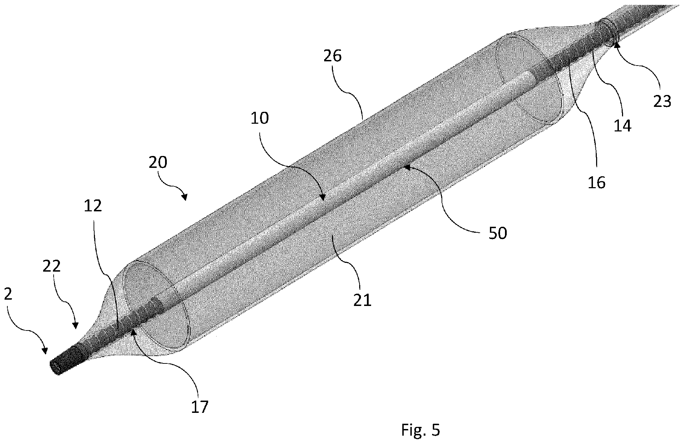

Fig. 5 shows in a perspective view a third embodiment of the distal part of the balloon catheter 1 with the distal tip 2. According to this embodiment, the radiopaque marker 50 is formed as a part of the outer layer 14 of the inner shaft 10. The outer layer 14 has at least partially radiopaque properties in a portion indicating the inner balloon lumen 21. This radiopaque marker 50 can be formed integral with the outer layer 14 by applying different materials forming the outer layer 14 onto the support body 16 and the inner layer 12. For the radiopaque marker 50 this material can be a particle-loaded radiopaque polymer meanwhile the other parts of the outer layer 14 is made of a polymer without radiopaque property. In this embodiment the radiopaque marker 50 may also have the same length as the cylindrical portion 26 of the inner balloon lumen 21, the same length as the length between the distal end portion 22 and the proximal end portion 23 or extends at least beyond one of these portions. The advantage of this embodiment is that the radiopaque filled polymer in combination with a coated or plated support body 16 and the overlying layer, enhances the radiopacity of the marker portions 50.

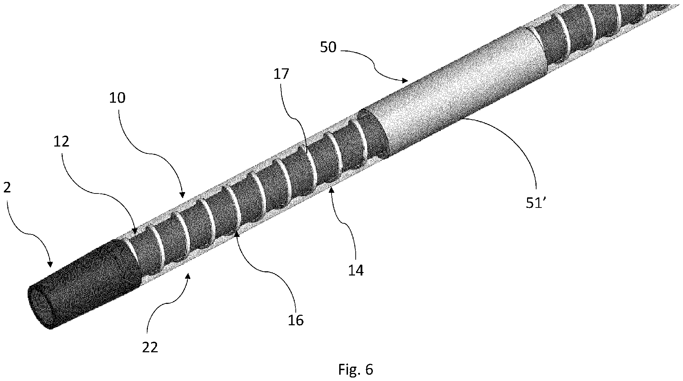

A fourth embodiment of the invention is shown in Fig. 6, whereby the balloon 20 is not shown explicitly. The radiopaque marker 50 is configured as a first radiopaque marker portion 51' located proximal to the distal end portion 22 and a second radiopaque marker portion 51" (not shown) located proximal to the proximal end portion 23 (not shown) of the balloon 20. These radiopaque marker portions 51', 51" could be made integral with the outer layer 14 by immersing parts of the inner layer 12 and the support body 16 into a polymer loaded with radiopaque particles and the other parts of the outer layer 14 are made by immersing the arrangement of inner layer 12 and support body 16 into a suitable polymer. Therefore, these radiopaque marker portions 51', 51" are embedded in the inner shaft 10 forming the outer layer 14 and therefore form a smooth outer surface of the inner shaft 10.

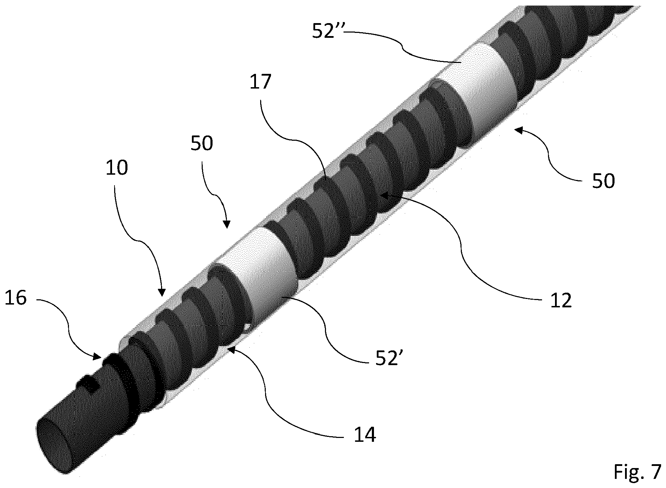

Fig. 7 shows in detail a fifth embodiment of the invention. According to this embodiment the radiopaque marker 50 is formed as a ring 52, in particular a first ring 52' and a second ring 52", which are located onto the support body 16, configured as the coil-shaped body 17. The first ring 52' and the second ring 52" have an inner diameter which is equal or slightly greater than the outer diameter of support body 16 arranged onto the outer surface of the inner layer 12 such that the first ring 52' and the second ring 52" can slip over the arranged support body 16 into the corresponding position along the length of the inner shaft 10. The first ring 52' and the second ring 52" are placed according to the size of the balloon 20. The first ring 52' and the second ring 52" can be made from a radiopaque metal such as but not limited to platinum, tungsten, gold, iridium, silver, and alloys thereof or radiopaque filled polymer like tungsten-filled polymer.

The first ring 52' and the second ring 52" are then embedded into the inner shaft 10 by applying the outer layer 14 onto the outer surface of the inner layer 12, the support body 16 and the positioned rings 52', 52". Therefore, the rings 52', 52" are located at a more inner position in the radial direction than the outer surface of the outer layer 14, in order to reduce the possibility of damaging of the balloon 20. With the first ring 52' and the second ring 52" located at predetermined positions, it is possible to confirm the position of the balloon 20 inserted into a vascular system.

While the invention has been described in detail with reference to specific embodiments thereof, it will be apparent to one skilled in the art that various changes and modification may be made, and equivalents thereof employed, without departing from the scope of the claims.

## Claims

1. A balloon catheter (1) for percutaneous transluminal angioplasty procedures comprising:
- an inflatable balloon (20) defining an inner balloon lumen (21) with a distal end portion (22), a cylindrical portion (26) and a proximal end portion (23),
- an inner shaft (10) passing through the inner balloon lumen (21), the inner shaft (10) including an inner layer (12), an outer layer (14), and a support body (16),
- the support body (16) is embedded in the outer layer (14) and extends over at least part of the inner shaft (10), the part being positioned within the inner balloon lumen (21), and
- at least a radiopaque marker (50) including a material with radiopaque property located within the inner shaft (10).

2. The balloon catheter (1) for percutaneous transluminal angioplasty procedures according to claim 1, wherein the support body (16) is a coil-shaped body (17) or a braid-shaped body.

3. The balloon catheter (1) for percutaneous transluminal angioplasty procedures according to claim 1 or 2, wherein the support body (16) extends at least over the cylindrical portion (26) of the balloon (20) or along almost the entire length of the inner shaft (10).

4. The balloon catheter (1) for percutaneous transluminal angioplasty procedures according to claim 2 or 3, wherein the coil-shaped body (17) is formed of a substantially rectangular wire.

5. The balloon catheter (1) for percutaneous transluminal angioplasty procedures according to claim 2 or 3, wherein the coil-shaped body (17) is formed of a round wire.

6. The balloon catheter (1) for percutaneous transluminal angioplasty procedures according to any one or more of the preceding claims, wherein part of the support body (16) is made of a material with radiopaque property forming the at least one radiopaque marker (50).

7. The balloon catheter (1) for percutaneous transluminal angioplasty procedures according to any one or more of the preceding claims, wherein at least one part of the outer layer (14) of the inner shaft (10) includes a radiopaque material forming the least one radiopaque marker (50).

8. The balloon catheter (1) for percutaneous transluminal angioplasty procedures according to any one or more of claims 1 to 5, wherein the at least one radiopaque marker (50) is formed by a coating (18) or plating of at least part of support body (16) with a material with radiopaque property.

9. The balloon catheter (1) for percutaneous transluminal angioplasty procedures according to any one or more of the preceding claims, wherein the radiopaque marker (50) extends over the entire cylindrical portion (26) of the inner balloon lumen (21).

10. The balloon catheter (1) for percutaneous transluminal angioplasty procedures according to any one or more of claims 1 to 5, wherein the at least one radiopaque marker (50) is configured as a ring (52) with an inner diameter equal or greater than an outer diameter of the support body (16) such as to be slipped over the support body (16) and embedded into the outer layer (14).

11. The balloon catheter (1) for percutaneous transluminal angioplasty procedures according to any one or more of the claims 1 to 8 and 10, wherein the radiopaque marker (50) is configured as a first radiopaque marker portion (51') located at a distal end of the cylindrical portion (26) of the inner balloon lumen (21) and a second radiopaque marker portion (51 ") located at a proximal end of the cylindrical portion (26) of the inner balloon lumen (21).

12. The balloon catheter (1) for percutaneous transluminal angioplasty procedures according to any one or more of the claims 1 to 8 and 10, wherein one or more radiopaque markers (50) are located either at a distal end of the cylindrical part (26) of the inner balloon lumen (21), at a proximal end of the cylindrical part (26) of the inner balloon lumen (21), at a centre of the cylindrical part (26) of the inner balloon lumen (21) or at any position within the cylindrical portion (26) of the inner balloon lumen (21).

13. The balloon catheter (1) for percutaneous transluminal angioplasty procedures according to any one or more of the preceding claims, wherein the material with radiopaque property is metallic and is selected from the group including platinum, gold, silver, iridium, tungsten, tantalum, and alloy thereof.

14. The balloon catheter (1) for percutaneous transluminal angioplasty procedures according to any one or more of the claims 1 to 12, wherein the material with radiopaque property includes a polymer with metal particles.

15. The balloon catheter (1) for percutaneous transluminal angioplasty procedures according to any one or more of the preceding claims, wherein the inner shaft (10) includes a distal end formed by a tapered distal tip (2), a guide wire lumen extending from at the distal end to a rapid exchange port at a proximal end of the inner shaft (10).
